# EUROPEAN PATENT SPECIFICATION

(11) **EP 3 531 996 B1**
(45) Date of publication and mention of the grant of the patent: **21.09.2022**
(21) Application number: 17866076.7
(22) Date of filing: 29.10.2017
(51) Int. Cl.: A61H 21/00, A61H 9/00, A61H 23/02, A61H 23/04, A61M 13/00, A61F 11/00

(54) **EAR THERAPEUTIC DEVICE**
THERAPEUTISCHE VORRICHTUNG FÜR DAS OHR
DISPOSITIF THÉRAPEUTIQUE POUR L'OREILLE

(30) Priority: 30.10.2016 US 201662414735 P
(43) Date of publication of application: 04.09.2019
(73) Proprietor: Yaadi Pituach Ltd., 68036 Tel-Aviv (IL)
(72) Inventor: AVNI, Yuval, 68036 Tel-Aviv (IL)
(74) Representative: Lecomte & Partners
(86) International application number: PCT/IL2017/051176
(87) International publication number: WO 2018/078630

(56) References cited:
- WO-A1-2013/139645
- WO-A1-2015/074060
- JP-A- 2009 022 699
- US-A- 2 099 871
- US-A- 4 896 679
- US-A1- 2002 069 883
- US-A1- 2010 002 897
- US-A1- 2013 237 746
- US-A1- 2016 067 099

## Description

The present invention generally relates to the field of ear devices, and more particularly, the present invention pertains to an ear device, providing technological solutions that simplify treatment of ear pain/discomfort, from such as Eustachian tube dysfunctions and barotrauma, during its operation and methods thereof, comprising a device operable in a plurality of treatment protocols and sub protocols of pressure and/or oscillating vibrations.

### BACKGROUND OF THE INVENTION

The ear, as a stato-acoustic organ has two major roles: hearing and maintaining equilibrium. The ear comprises three different portions, the outer, middle, or inner ear. The **outer ear** is the external portion of the ear, which consists of the pinna and external auditory meatus. It gathers sound energy and focuses it on the eardrum. From the pinna the sound pressure waves move into the ear canal, also known as the *external acoustic meatus* a simple tube running through the middle ear. The **middle ear** is the portion of the ear internal to the eardrum, and external to the oval window of the inner ear. The middle ear contains three ossicles, which transfers the vibrations of the eardrum into waves in the fluid and membranes of the inner ear. The hollow space of the middle ear has also been called the tympanic cavity, or *cavum tympani.* The Eustachian tube joins the tympanic cavity with the nasal cavity (nasopharynx), allowing pressure to equalize between the middle ear and throat. The Eustachian tube originates in the rear of the nose adjacent to the soft palate, runs a slightly uphill course, and ends in the middle ear space. The middle ear space is the hollowed out portion of the skull bone that contains the hearing apparatus and is covered on one side by the eardrum. The **inner ear** (auris interna) is the innermost part of the vertebrate ear. It is mainly responsible for sound detection and balance. It consists of the bony labyrinth, comprising two main functional parts: the cochlea, dedicated to hearing; converting sound pressure patterns from the outer ear into electrochemical impulses which are passed on to the brain via the auditory nerve, and the vestibular system, dedicated to balance.

Otalgia, ear pain, is a very common symptom of various conditions and diseases. It can originate from the different portions of the ear, or referred to from an external location such as from the jaw area.

Two of the most prominent pain symptoms are related to the Middle ear. These include pain originating from mechanical conditions such as Eustachian tube obstruction, barotrauma, or inflammatory conditions originating in infection. Ear infections can occur as side effects of contagious illnesses-colds, coughs, or eye ailments. The tissue that lines the Eustachian tube is similar to that inside the nasal cavity and may respond the same way (swelling) when presented with similar stimuli.

The main functions of the Eustachian tube, as known in the art are: to ventilate the middle ear space, ensuring that its pressure remains at near normal environmental air pressure, and to drain any accumulated secretions, infection, or debris from the middle ear space. Several small muscles located in the back of the throat and palate control the opening and closing of the tube. Swallowing and yawning cause contractions of these muscles located in the back of the throat and help regulate Eustachian tube function. Normally, the Eustachian tube is closed, which helps prevent the inadvertent contamination of the middle ear space by the normal secretions found in the back of the nose.

Eustachian tube dysfunction (ETD) occurs when the tube fails to open during swallowing or yawning. This results in a difference between the air pressure inside and outside the middle ear. ETD is caused by poor function or blockage of the Eustachian tube, including: inability of the tiny hairs inside the ear to remove fluid and infection; poor squeezing function within the Eustachian tube; narrow Eustachian tube in infants; adenoid tissue blocking Eustachian tube in children; swollen nasal secretions that cause a blockage; and, tumors in adults.

Factors that may increase your chance of getting ETD include: age (children), nasal congestion from an allergy, cold other upper respiratory infection, ear or sinus infections, environmental allergies, children with large adenoids, activities with large rapid altitude changes (flying or scuba diving), and presence of obstructing tumors in the nasopharynx. Symptoms can include: feeling of fullness or clogging in the ear, discomfort or ear pain, hearing loss, ear ringing, a sensation of spinning known as vertigo, pain if the blockage results in an infection.

Ear barotrauma is discomfort and possible damage in the ear due to pressure differences between the inside and outside of the eardrum. Barotrauma can originate from the common problem of failure of the Eustachian tube to effectively regulate air pressure. Partial or complete blockage of the Eustachian tube, whether cold related or constructional abnormality, can cause sensations of popping, clicking, and ear fullness and occasionally moderate to severe ear pain. Such intense pain is most frequently experienced during sudden air pressure changes during airplane travel, scuba diving, or driving in the mountains. As air pressure in the middle ear falls, and the ear feels full and sounds are perceived as muffled. Eventually, a vacuum is created which can then cause fluid to be drawn into the middle ear space (termed serous otitis media). If the fluid becomes infected, the common ear infection develops. Other symptoms include dizziness, hear loss (from slight to severe), ear discomfort and nose bleed.

JP 2009-022699 discloses an ear canal massager formed into a shape which does not allow entrance into the deep location of the external acoustic meatus even when the massager is firmly pressed to the ear. Also, grooves are applied on the surface of the massager, or a ventilation path is provided inside so that the difference in the air pressures may not be generated on the inside and the outside of the external auditory meatus which is closed by the massager. Alternatively, the massager is formed of a material which can permeate air, or formed into a structure which is deformed at the time of use.

WO 2015/074060 A1 discloses a device for stimulating at least one cranial nerve and/or spinal nerve is described. The device includes a vibratory motor, and an earpiece, wherein the earpiece is molded substantially to fit within the external ear canal and contacting the concha of a subject's ear. A method of reducing migraine headache and trigeminal neuropathy pain is also described. The method includes positioning a vibratory earpiece within an ear of a subject, applying vibrational energy to at least a portion of the skin of at least one of the auditory canal, auricle and concha of the ear, thereby stimulating at least one sensory fiber of at least one of cranial nerve 5, cranial nerve 7, cranial nerve 9, cranial nerve 10, spinal nerve C2, and spinal nerve C3. A method to normalize breathing, normalize blood pressure, induce sleep, increase salivation, and improve vertigo, nausea, and visual dysfunction accompanying migraine is also described.

Therefore, there is a long felt and unmet need for a device that provides pain relief and aids in recovery of middle ear related conditions.

### SUMMARY OF THE INVENTION

The present invention provides an ear therapeutic device ETD according to claim 1.

Further developments are according to the dependent claims 2-6.

### BRIEF DESCRIPTION OF THE FIGURES

In the following detailed description of the preferred embodiments, reference is made to the accompanying drawings that form a part hereof, and in which are shown by way of illustration specific embodiments in which the invention may be practiced. It is understood that other embodiments may be utilized and structural changes may be made without departing from the scope of the present invention. The present invention may be practiced according to the claims without some or all of these specific details. For the purpose of clarity, technical material that is known in the technical fields related to the invention has not been described in detail so that the present invention is not unnecessarily obscured. In the accompanying drawing:
**Fig. 1** is a schematic illustration of an example of the ear therapeutic device.
**Fig. 2** is a schematic illustration, in an out of scale manner of various ear drum positions.
**Fig. 3A-3B** are schematic, out of scale, illustration of embodiments of a mouth piece of the ear therapeutic device of the invention.
**Fig. 4A** is a schematic illustration, in an out of scale manner of an example of an ear therapeutic device for self-use.
**Fig. 4B** is a schematic illustration, in an out of scale manner of an example of an ear therapeutic device for operation by a care taker.
**Fig. 4C** is a schematic illustration, in an out of scale manner of an example of an ear device having a flexible portion and a turnable hinge.
**Fig. 5A-B** are schematic illustrations, in an out of scale manner of different examples of the invention comprising a nose mask.
**Fig. 5C-D** are schematic illustrations, in an out of scale manner of different examples comprising a nostril apparatus.
**Fig. 6A** is a schematic illustration, in an out of scale manner of an embodiment of the invention comprising a pressure control device.
**Fig. 6B-D** are schematic illustrations, in an out of scale manner of various embodiments of the pressure controller.
**Fig. 7A-B** are schematic illustrations, in an out of scale manner of various examples of pressure providing means.
**Fig. 8** is a schematic illustration, in an out of scale manner of an example comprising a conduit having three apertures, useful for providing pressure to the Eustachian tube from the ear connecting side and from the throat connecting side via the nasal cavity.
**Fig. 9A** is a schematic illustration, in an out of scale manner of an example worn by a patient, and connected to the patient's mouth and ear.
**Fig. 9B** is a schematic illustration, in an out of scale manner of an example worn by a patient, and connected to the patient's mouth, nose and ear.
**Fig. 10A** is a schematic illustration, in an out of scale manner of an embodiment of the present invention operable by the patient's inhaling and exhaling, comprising an earphone.
**Fig. 10B** is a schematic illustration, in an out of scale manner of an embodiment of the present invention, operable by the patient's inhaling and exhaling, comprising an at least partial facial cover.
**Fig. 11A-B** is a schematic illustration, in an out of scale manner of an embodiment of the invention comprising inhalation and exhalation valves.
**Fig. 12 A-B** are schematic illustrations, in an out of scale manner of different examples comprising an ear vibrator.
**Fig. 13** is a schematic illustration, in an out of scale manner of an example, comprising pneumatic pressure means and vibration means.
**Fig. 14 A-C** are schematic illustrations, in an out of scale manner of different examples comprising an inflatable membrane and oscillating vibration providing means.
**Fig. 15** is a schematic illustration, in an out of scale manner of an example in a side view, showing an example of a user interface.
**Fig. 16A-B** are schematic illustrations, in an out of scale manner of different embodiments of the invention comprising a first inflatable membrane for providing alternating pressure, a second inflatable membrane providing ear canal sealing and pressure control, and oscillating vibration providing means.
**Fig. 17** is a schematic illustration, in an out of scale manner of an example comprising a remote control system.

### DETAILED DESCRIPTION OF THE PREFERRED EMBODIMENTS

In the following detailed description of examples and preferred embodiments, reference is made to the accompanying drawings that form a part hereof, and in which are shown by way of illustration specific embodiments in which the invention may be practiced. It is understood that other embodiments may be utilized and structural changes may be made without departing from the scope of the present invention. The present invention may be practiced according to the claims without some or all of these specific details. For the purpose of clarity, technical material that is known in the technical fields related to the invention has not been described in detail so that the present invention is not unnecessarily obscured.

The essence of the present invention is to provide an ear therapeutic device intended for relieving pain and/ or discomfort of the ear by balancing pressure in the middle ear. This is achieved by delivering pneumatic pressure and/or oscillating vibrations via the ear canal, the nasal cavity, and/or other head tissue, relieving positive/negative ear pressure and assisting in fluid drainage during its operation and methods thereof.

The term **"ear piece"** interchangeably refers hereinafter to any member, part, or portion of the ear device of the present invention that is configured to at least partially interface with at least a portion of the patient's ear. The ear piece can be as such as an earphone, ear cover, ear insert, ear muff and etc. Further the ear piece can be at least partially inserted into the ear, at least partially on the ear, at least partially around the ear, at least partially hanging on at least a portion of the ear, supported by a head / neck / shoulder connecting support (such as a bow, elastic strap, sticker, band, rigid support, and etc.), and any combination thereof. Additionally or alternatively, the ear piece is configured to at least partially block or seal the ear external canal while allowing the passage of pneumatic pressure in the form of movement of air, an inflated balloon and/or expandable membrane. Additionally or alternatively, at least a portion of the ear piece is comprised of sterilizable material, or disposable material, and may be reversibly attachable and detachable from the device. Additionally or alternatively, at least a portion of the ear piece is made of materials comfortable to ware such as padded elements, rubber elements and etc. Additionally or alternatively, the ear piece further comprises at least one portion configure to be at least partially inserted into the external ear canal and optionally at least one adjustable portion fitting to the outer ear.

The term **"mouth piece"** or **"mouthpiece"** interchangeably refers hereinafter to a part of the device of the present invention which comes near or in contact with one's mouth during use. The mouthpiece can be of various embodiments, such as, as non-limiting examples: a simple opening that leads to the main body of the device of the present invention, a mouth piece comprising at least a portion partially protruding configured to be at least partially insertable into a user's mouth, a mouthpiece is a part having at least a portion thereof configured to be at least partially gripped by the user grips in his/her mouth, a mouthpiece comprising an outer rubber flange that fits outside the lips having a mouth mask like formation, and any combination thereof.

The term "**nose piece"** interchangeably refers herein to any device configured to at least partially seal at least a portion of the patient nasal cavity and allow administration of pneumatic pressure thereto. Additionally or alternatively the nose piece is such as a mask around the nose, on the nose, at least partially insertable to at least one nostril, and any combination thereof. It is in the scope of the invention to provide pneumatic pressure to the patient nasal cavity thereby assisting in closure of the patient soft palate.

The term **"flexible material"** interchangeably refers hereinafter to any material capable of bending easily without breaking, or that is able to be easily modified to respond to altered circumstances or conditions. This can be as a none-limiting example: rubber, synthetic rubber, silicon, polyamides, nylon, textile, cloth, plastic, polyester, polyethylene, polyvinyl chloride (PVC), low-density polyethylene (LDPE), polypropylene, polyurethanes, polysulfone, and any composite materials of the any of the above.

The term **"vibration means"** refers hereinafter to any device, apparatus, instrument assembly, able to produce artificially induced vibrations, harmonic or none harmonic. As a none-limiting example, this can be such as a vibrator, eccentric weights, vibration piston, acoustic generator, servohydraulic (electrohydraulic) shakers, electrodynamic shakers, and etc. The vibrations improve mucociliary clearance of secretions in Eustachian tube and airways, thus preventing accumulation of secretions and diseases.

The term **"pressure means"** interchangeably refers hereinafter to any pressure equalizing means, including pneumatic pressure application by such as: a user's air blowing, a pneumatic pump, bellow, piston, an electrical pneumatic pump, a manual pump, an air compressor, ventilator, fan, flexible bag, and etc. The pressure applied by the system can be positive, negative or both. Negative pressure can be achieved by vacuum producing means. Pressure producing means can be covered by an inflatable / deflatable balloon or flexible membrane which provides a selectable overall or basal pressure.

The term **"data logger"** interchangeably refers hereinafter to any instrument having a vibration / pressure sensor and a digital memory, able to measure, record and store autonomously shocks, vibrations, or pressure over a defined period of time. Digital data is usually in the form of acceleration, amplitude and time. The data can be retrieved, viewed and evaluated after it has been recorded.

The term **"external environment"** refers hereinafter to the external space outside of the patient's body.

The term **"about"** refers hereinafter to 20% more or less than the defied value.

The term **"patient"** interchangeably refers herein after to a term selected from a group of: neonate, baby, infant, toddler, child, adolescent, adult, elderly, etc.; further this term refers to person or animal.

The term "user" interchangeably refers herein to any person operating the ear therapeutic device. This can be either the patient, or any care taker.

The term **"transparent material"** interchangeably refers hereinafter to materials such as, polymethyl methacrylate, thermoplastic polyurethane, polyethylene, polyethylene terephthalate, isophthalic acid modified polyethylene terephthalate, glycol modified polyethylene terephthalate, polypropylene, polystyrene, acrylic, polyacetate, cellulose acetate, polycarbonate, nylon, glass, polyvinyl chloride, etc. Further in some embodiments at least a portion of this material is imbedded with non-transparent materials for means of strength and/ or conductivity such as metallic wires.

The term **"conduit"** interchangeably refers to any at least partially hollow construct configured to allow fluid communication therethrough. This can be such as tube, channel, canal, hose, line, duct, pipe, cable, vein, route, aisle, aqueduct and etc. additionally or alternatively, at least a portion of the conduit is made of flexible material, rigid material or both. Additionally or alternatively, the conduit comprises one or more openings. Additionally or alternatively the conduit is branched into two or more conduits.

The term **"patient's monitoring sensor**" interchangeably refers to any sensor monitoring the physical condition of the patient. The sensor can be, as a none-limiting example, a cardio vascular sensor, a breathing sensor, a pressure sensor in the ear, a temperature sensor, a movement sensor, a liquid sensor for the middle ear, and etc.

It is disclosed to provide **"protocols"** and "**sub protocols**" that include vibration producing oscillations, pneumatic pressure, or both, administered individually or simultaneously. The protocols are comprised of any combination of the mentioned sub protocols.

Both the vibrations and pneumatic pressure are administered with sup protocols including constant administration, intermittent, pulsating, or any combination thereof.

Sub protocols for providing different pressures having at least one variable parameter such as amplitude, duration of velocity of pressure incline or decline, pulsating or constant, frequency of pulsation, wave length, and different wave forms. It is disclosed to provide a pressure wave that of a form such as sinusoid, triangular, rectangular, trapezoid, linear, curved, arched, or any other wave form symmetric or non-symmetric. Additionally or alternatively, the wave is pulsating, and/or pulsating in an attenuated, intensified and any combination thereof, manner. Additionally or alternatively, the administered pressure is positive, negative or a combination sequence of both.

Sub protocols providing different vibrations include administering vibrations such as:
Vibration from one or more vibration means, applying vibrations to one or more locations, individually or simultaneously. The vibrations can be humming vibrations, oscillating vibrations, acoustical originating vibrations, mechanical originating vibrations, and etc.

A plurality of vibrations can be administered in the sub protocols comprising at least one variable parameter such as waveform, duration, wave length, frequency, amplitude, velocity, direction, and any combination thereof. It is disclosed to provide a vibration wave that of a form such as sinusoid, triangular, rectangular, trapezoid, linear, or any other wave form symmetric or non-symmetric. Additionally or alternatively, the wave is pulsating, and/or pulsating in an attenuated, intensified and any combination thereof, manner.

There may be more than one effector, for example, for producing vibrations within a particular range of amplitudes and frequencies, whereas other effectors may be adapted to produce vibrations within other ranges of amplitudes and frequencies. The mechanical vibrations provided may be applied directly to the patient's affected part, or they may be transmitted through inflated balloons, pads or cushions.

Sub protocols providing different pressure durations, and different protocol durations

It is disclosed to provide **"protocols"** and **"sub protocols"** defined by a medical care taker or doctor, predefined by the manufacturer, manually configured by the user/ patient, or providing personalized treatment of the patient, according to his/ her specific needs. These can be tailored individually to each patient.

It is disclosed to provide the device as defined above, wherein the protocols include silent periods and active periods as herein described.

Other optional elements may be provided, including: heating elements, and fluid perfusion or medicated fluid administration means.

The term **"visual indicators"** interchangeably refers hereinafter to a representation of light in the visible light range of about 380 nanometers to about 740 nm. More generally the terms refer to any light within the visible range that will be noticeable by the user of the invention (light, flashing light, flickering light, blinking light, change of spectrum of colors of light etc.).

The term **"audible indicators"** interchangeably refers hereinafter to a representation of sound, typically as an electrical voltage. Audible indicators have frequencies in the audio frequency range of roughly 20 to 20,000 Hz (the limits of human hearing). Audible indicators are either synthesized directly, or originate at a transducer such as a microphone, musical instrument pickup, phonograph cartridge, or tape head.

The term **"sensible indicators"** interchangeably refers hereinafter to a physical movement of at least a portion of the user interface, which is noticeable to the user (shaking, vibrating, quivering, etc.).

The term **"connected"** in reference to the ear therapeutic device, and ear therapeutic device parts and modules, interchangeably refers hereinafter to any contact, relation, association, integration, interconnection, joining, inserting, sewing, welding, interweaving, placing, nesting, layering, etc., of the ear device parts to each other and to a third party.

The term **"plurality"** interchangeably refers hereinafter to an integer *a,* when *a* > 1.

The term **"manual"** in respect to the ear therapeutic device interchangeably refers herein after to any application of force by the handler aimed at moving at least a portion of the device. This force is generated by an action such as pushing, pulling, lifting, levering, turning, twisting, hitting, lowering, air blowing, inhaling, exhaling, pressing, tilting and etc.

The term **"emergency release mechanism",** interchangeably refers hereinafter to a mechanism used in immediate need arrest of the device. This could be in case of malfunction, fire, excess heating, patient discomfort, patient trauma or medical condition, technical difficulty and etc. The emergency release can be operated manually by the patient or care taker, or by a predefined set of terms, such as: the temperature sensed by an attached temperature sensor exceeds a predefined valve, the pressure sensed by a pressure sensor exceeds a predefined value. Medical sensors monitoring the physical conditions of the patient at below or above a predefined range, and etc.

The term **"Computer Readable Media",** (CRM), interchangeably refers hereinafter to, a medium capable of storing data in a format readable by a mechanical device (automated data medium rather than human readable). Examples of machine-readable media include magnetic media such as magnetic disks, cards, tapes, and drums, punched cards and paper tapes, optical disks, barcodes and magnetic ink characters. Common machine-readable technologies include magnetic recording, processing waveforms, and barcodes. Optical character recognition (OCR) can be used to enable machines to read information available to humans. Any information retrievable by any form of energy can be machine-readable.

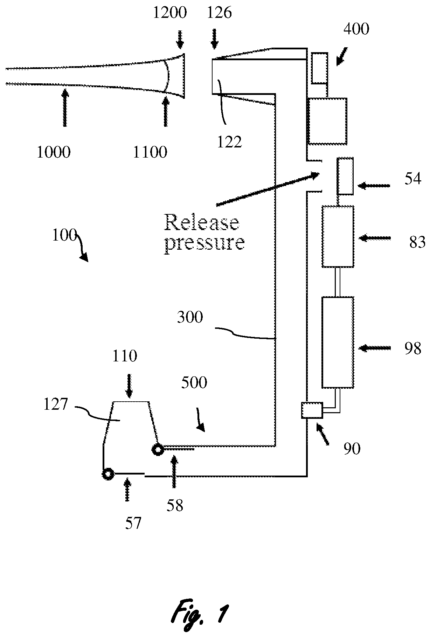

Reference is now made to **Fig. 1** schematically illustrating, in an out of scale manner, an example of the ear therapeutic device (100). Ear pressure equalizing means (500) comprising at least one conduit (300) having at least one first end attachable to the ear aperture (126), and at least one second end (127) connected to the mouth aperture. At least one first conduit end comprises an ear piece (122), and at least one second ear piece is connected to a mouth piece (110). The conduit (300) is configured to permit the passage of air from the mouth piece (110) to the ear piece (122). The ear piece is configured to at least partially seal the external ear canal (1200), while allowing pneumatic pressure to pass through enabling application of positive or negative pressure on the ear drum (1100). The conduit (300) is further connected to a controller (98) designed to control the pneumatic pressure applied by the device (100). The controller is further connected to a release valve motor (83) and at least one release valve (54) configured to allow pressure release and pressure buildup when opened or closed accordingly. The pressured is also monitored by at least one pressure sensor (90), relaying the sensed information to such as the controller, at least one indicator, or a CRM. The device further comprises at least one vibrator (400), configured to provide oscillating vibrations to the ear by the earphone. The vibrations can improve draining of fluids in the ear and the Eustachian tube (1000), promoting healing and /or pressure equalizing.

Additionally or alternatively the device can be operated by the patient inhaling, exhaling, forcibly blowing air, by remote control, by a wireless/ wired connection to a remote computer, by the controller having a user interface including operational features such as buttons, knobs, sliders, touch screen, keyboard, voice activation, computer mouse, and etc. Additionally or alternatively, the device (100) is configured to operate by a providing a treatment protocol of vibrations and/ or pneumatic pressure. The device (100) is configured to provide at least one protocol of administering at least one of the following: vibrations, positive pressure, and negative pressure. This administration is either simultaneously or intermittently or a combination of both. Additionally or alternatively the time duration of each protocol or sub protocol can be predefined, manually changed by the user, or both. Additionally or alternatively the specific treatment protocol can be predefined, manually adjusted by the user or both. Further, the unique combination of protocols and sub protocols can be predefined or manually adjusted according to such as the patient input, the directions of medical personal, and etc. Additionally or alternatively, a plurality of sub-protocols is provided comprising at least one set of vibrations, having differential wavelength, frequency, amplitude, velocity, duration, waveform, and any combination thereof. Any set of vibrations can be provided in combination with pneumatic pressure. The pneumatic pressure can be either uniform or variable, constant or intermitted and any combination thereof, and can also be administered in an oscillating wave formation. Additionally or alternatively, the pneumatic pressure is administered in a protocol comprising any combination of pneumatic pressure and vibration. Further, the vibration can be applied by one or more vibrators. When comprising a plurality of vibrators, the vectors of vibrations can be parallel, intersecting, unparalleled, divergent or similar. Additionally or alternatively, a sub-protocol is provided configured to administering vibrations from different at least partially intersecting vectors forming new combinations of vibrations due to wave interference.

The mouth piece is provided with at least one inhalation valve (57) and at least one exhalation valve (58). When inhaling, the inhalation valve (57) opens to allow the entry of external air through the mouthpiece into the patient's mouth, and close to prevent the escape of air, and consequence pressure decline when exhaling. In an example, the exhalation valve remains closed such that mainly external air reaches the mouth piece. When exhaling the exhalation valve maneuvers to an open position allowing the air originating at the patient's mouth to pass towards the earphone.

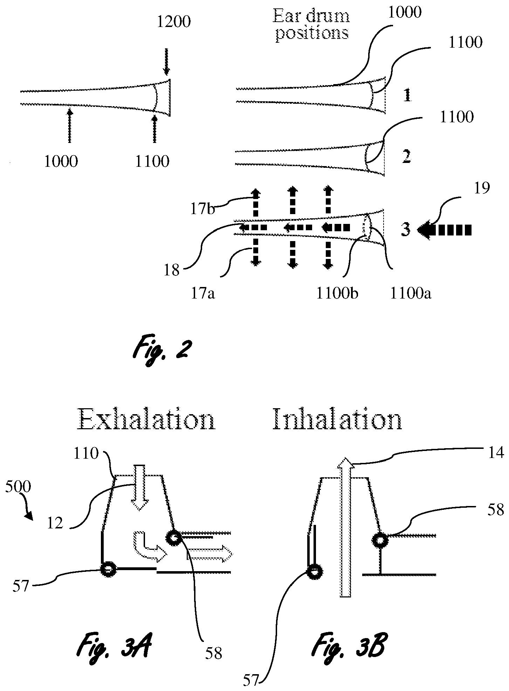

Reference is now made to **Fig. 2**, schematically illustrating, in an out of scale manner, various ear drum (1100) positions. A portion of the ear is schematically drawn to exemplify the Eustachian tube (1000), the ear drum (1100) and the external ear canal (1200). **Fig. 2** (1) shows the position of the ear drum (1100) when internal positive pressure is applied forcing a protrusion of the eardrum towards the ear canal. **Fig. 2** (2) shows another position of the ear drum when negative pressure is formed within the ear (positive pressure from the external ear canal) and as a result, the eardrum is protruding towards the inner ear.

Both these situations are associated with patient discomfort, and are usually equalized by temporarily opening of the Eustachian tube. When unable to do so due to for example a clog of the Eustachian tube (caused by such as cold, allergy, infection and etc.), the patient may experience dizziness, pain, hearing impairment, stuffiness. **Fig. 2** (3) shows the direction of pressure and / or vibration application to the ear via the external ear canal affecting the ear drum to maneuver from formation 1100a to formation 1100b and the vibrations to travel along the Eustachian tube, as an none limiting example, in the direction indicated by the arrows (17a, 17b, 18).

Reference is now made to **Fig. 3A-B** schematically illustrating, in an out of scale manner, embodiments of a mouth piece (110) of the ear therapeutic device of the invention. The mouth piece is provided with at least one inhalation valve (57) and at least one exhalation valve (58). **Fig. 3B** illustrates an example of the valves position when inhaling. When inhaling, the inhalation valve (57) opens to allow the entry of external air (14) through the mouthpiece into the patient's mouth, and close to prevent the escape of air, and consequence pressure decline when exhaling. In an embodiment, the exhalation valve (58) remains closed such that mainly external air reaches the mouth piece. **Fig. 3A** illustrates the position of the valves when exhaling. When exhaling the exhalation valve (58) maneuvers to an open position allowing the air originating at the patient's mouth to pass towards the earphone.

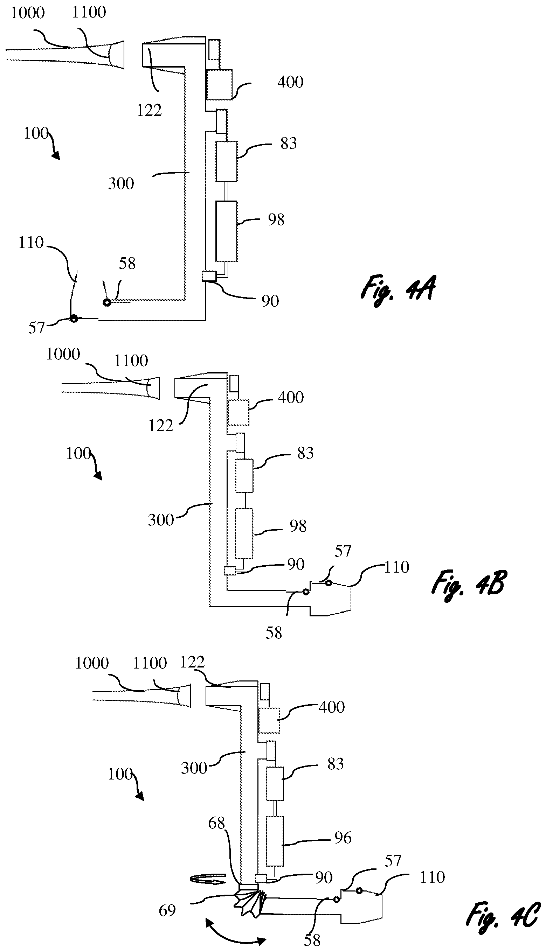

Reference is now made to **Fig. 4A**, schematically illustrating, in an out of scale manner of an example of an ear therapeutic device (100) for self-use. In this example, the conduit (300) is connected at one end to an ear piece/ ear phone (122), and on the other end to a mouth piece (110). The ear piece (122) is protruding in a substantially perpendicular angle to the conduit, and the mouth piece is connected to a parallel protrusion at the opposite end. This example enables the patient to use the device independently, unassisted. The device is configured such that when the ear piece (122) is at least partially interfacing with the patient's ear positioned towards the ear drum (1100) and the Eustachian canal (1000), the mouthpiece (110) reaches the patient's mouth. Additionally or alternatively, at least a portion of the conduit (300) is made from flexible materials enabling adjustments of the conduit such that the different parts and portions fit the patient. Additionally or alternatively, the conduit comprises at least a portion of a segmented structure. In this example, at least one segment is reversibly connectable to said conduit, such that this segment can be exchangeable with different sized or shaped segment such that the device is fitted to be used by different sized individuals. Additionally or alternatively, a different sized and / or shaped segment is used for the optional connection of additional vibration means, pneumatic pressure means, heating means, and any combination thereof. The device comprises at least one vibrator (400), at least one controller (98) connected to a release valve motor (83), operative to release a valve (54) according to information received by a pressure sensor (90). The ear device further comprises at least one inhalation valve (57) and at least one exhalation valve (58).

Reference is now made to **Fig. 4B** schematically illustrating, in an out of scale manner, an example of an ear therapeutic device for operation by a care taker. In this example the protrusion connecting the conduit (300) to the mouth piece (110) extends to substantially the opposite direction than the one comprising the ear piece. In this manner, easy access is provided for a care taker to operate the mouth piece (110) while the ear piece (122) is interfacing the patient. Reference is now made to **Fig. 4C**, schematically illustrating, in an out of scale manner, an example of the ear therapeutic device having an adjustable flexible portion (69) and a turnable hinge (68). Additionally or alternatively, the device comprises a plurality of flexible portions and/ or hinges. In this example the device can be operated by either the patient and/ or alternatively by a care taker. This, by adjusting the precise position and angle of different portions of the conduit utilizing the flexible portion and or one or more hinges.

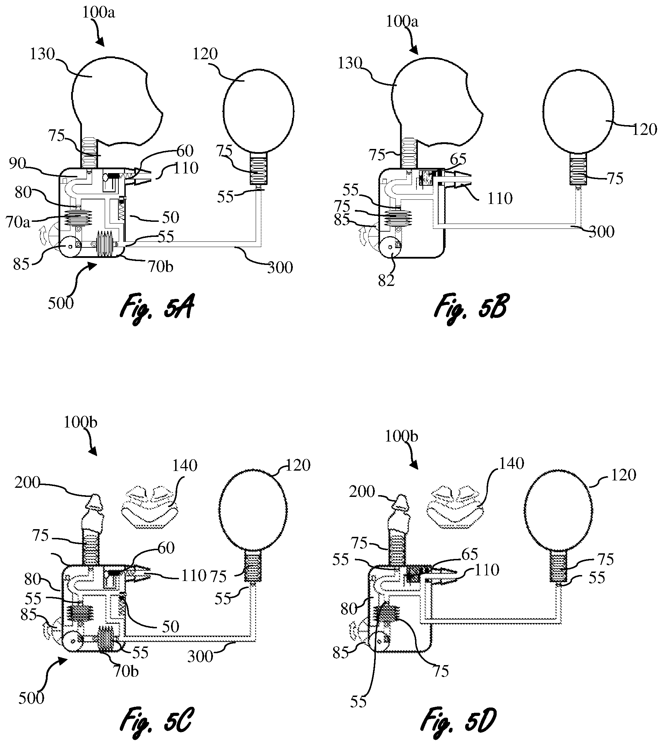

Reference is now made to **Fig. 5A-B,** schematically illustrating, in an out of scale manner different examples of the invention, comprising a nose mask (130). **Fig 5A** illustrates the ear therapeutic device (100a), comprising a conduit (300) embodied as a hollow tubing system configured to permit the passage of fluid within. In this example, the pneumatic pressure is provided by at least one motor (80) operating a plurality of pressure providing means such as at least one pump, bellow, piston, (70a, 70b), included in the pressure equalizing means (500). The conduit comprises tubing connecting one pump (70a) to a nose mask (130) and another pump is connected to an ear cover (120). The pneumatic pressure provided by the pumps is controlled by at least one of the following: a motor velocity adjustor (85), enabling at turning up /down the velocity of the motor, thereby adjusting the pressure buildup and the pressure buildup speed; one or more valves (50) positioned along the conduit; additional pressure providing means such as a pump, piston, and/or bellow (75) connected in proximity to the nose mask (130) and or ear cover (120); additional valves (55) can be placed at the airways in the pump exits. Additionally or alternatively, one or more of the valves is such as a servo valve, predefined to close/ open according to preset pressure values. Optionally, the valves are opened and/ or closed in accordance to a treatment protocol or sub-protocol. In addition, the user can control at least one valve (50) manually. The device (100a) is configured to be operated by the user inhaling, exhaling, and/ or air blowing into a mouth piece (110). In this example the mouth piece is connected to an on / off button (60) and a pressure control valve (50) configured to respond to the user's exhaling, and/ or air blowing. Additionally or alternatively, the device (100) is configured to administer pressure in the nasal pathways thereby assisting in closing of the soft palate. Reference is now made to **Fig. 5B**, schematically illustrating, in an out of scale manner, an example of the ear therapeutic device comprising one pneumatic pump in fluid communication with both the nose mask (130) and the ear cover (120). The pressure on/off is controlled by the user's inhaling / exhaling / air blowing, while the pressure applied is regulated by a pressure control device (65)

**Fig. 5C-D** are schematic illustrations, in an out of scale manner of different examples of the invention comprising a nostril apparatus (200). **Fig. 5C** illustrates the ear therapeutic device (100a), comprising a conduit (300) embodied as a hollow tubing system configured to permit the passage of fluid within. In this example, the pneumatic pressure is provided by at least one motor (80) operating a plurality of pneumatic pumps (70a, 70b). The conduit comprises tubing connecting one pump (70a) to an ear cover (120) and another pump is connected to nostril at least a partially fitting insert (200). Additionally or alternatively, the nostril piece is reversibly replaceable with a nose piece fitting at least a portion of both nostrils (140). The pneumatic pressure provided by the pumps is controlled by at least one of the following: a motor velocity adjustor (85), enabling turning up /down the velocity of the motor, thereby adjusting the pressure buildup and the pressure buildup speed; one or more valves (50, 55) positioned along the conduit; additional pumps (75) connected in proximity to the nose mask (130) and/ or ear cover (120); additional valves (55) can be placed at the airways in the pump exits. Additionally or alternatively, one or more of the valves is such as servo valve, predefined to close/ open according to preset pressure values. Optionally, the valves are opened and/ or closed in accordance to a treatment protocol or sub-protocol. In addition, the user can control at least one valve (50) manually. The device (100a) is configured to be operated by the user inhaling, exhaling, and/ or air blowing into a mouth piece (110). In this example the mouth piece is connected to an on / off button (60) and a pressure control valve (50) configured to respond to the user's exhaling, and/ or air blowing. Reference is now made to **Fig. 5D**, schematically illustrating, in an out of scale manner, an example of the ear therapeutic device comprising one pneumatic pump in fluid communication with both the nose piece (200), reversibly replaceable with a two nostril piece (140), and the ear cover (120). The pressure on/off is controlled by the user's inhaling / exhaling / air blowing, while the pressure applied is regulated by a pressure control device (65).

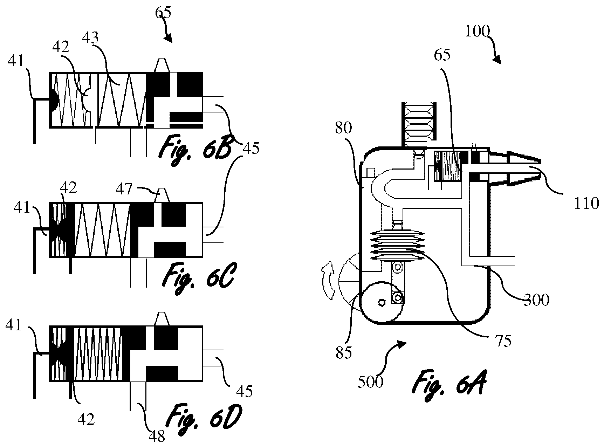

Reference is now made to **Fig. 6A**, schematically illustrating, in an out of scale manner an embodiment of the ear therapeutic device (100), including ear equalizing pressure means (500), and comprising a pressure regulating device (65). A motor (80) is configured to operate a pump (75) to apply fluid pressure through a tubing system (300). The pressure is controlled by a motor velocity controller slider (85), and/or by a pressure control device (85), connected to the tubing (300) adjacent to the mouth piece (110). The pressure control device is operable by the patient's / user applying positive or negative pressure by forcibly blowing air or forcibly inhaling. Additionally or alternatively, the device can be controlled by the patient's / user breathing velocity, pressure, time span of inhaling/ exhaling or both, inhaling, exhaling, repeated breathing sequence and etc.

Reference is now made to **Fig. 6B-D** schematically illustrating, in an out of scale manner various embodiments of the pressure controller (65). The fluid is passed through a conduit (300) and enter the pressure control device through an entry port (45), and exit through an exit port (48). The device further comprises at least one pressure release valve (47) configured to regulate the pressure applied. The pressure control device responds to the fluid pressure passing through by moving a slide-able portion (42) accordingly, towards a contact element (41). The pressure providing means is configured to be normally closed. When the slide-able portion connects to the contact element (41) the device is turned on. When the pressure rises, a spring (43), connecting the slide-able element to the contact element, condenses further generating a response of application of higher pressure administration.

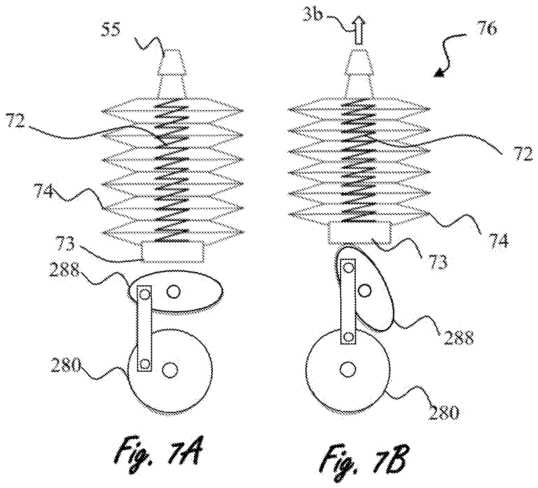

**Reference is now made to** **Fig. 7A-B** schematically illustrating, in an out of scale manner, an example of pressure providing means (76). This example can be, but not limited to the pressure providing means described in Fig. 5 (75, 70b, 70a). A motor is connected to a motor gear (280) transforming the rotating power of the motor to an up and down movement of an eccentric mechanism (288). The movement of the eccentric mechanism (288) provides intermittent contact between the bellow (74) and the force generated from the motor and transmitted by the motor gear (280). Additionally or alternativlley, the bellow (74) is a manual pump, automated pump, piston, air compressor, flexible bag, vent or any combination thereof. The term "Eccentric" or "**excenter**" interchangeably refers hereinafter to a mechanism that converts rotary motion into reciprocating motion where the eccentric diameter is larger than the eccentricity. Further, the circular eccentric disc is rotating about a point that does not coincide with the axis center. When the motor rotates, the motor gear (280) translates the rotating motion by the eccentric mechanism (288) to an intermittent movement causing the excenter piece (288) to apply a sharp blow to the base (73) of the bellow, thereby compressing the bellow and therefore providing a positive pressure wave in the direction of arrow 3b. Following, the internal return spring (72) expands the bellow back to its original form thereby providing negative pressure. In addition the air exits through a nozzle (55), which in some examples can be provided with a valve useful for regulating the pressure passing through the nozzle. **Fig. 7a** illustrates the bellow (74) in an expanded position, while the eccentric mechanism (288) is not touching the bellow base (73) and the return spring (72) in a normally open form (expanded). **Fig. 7b** illustrates the bellow (74) in a condensed position, following contact by the eccentric mechanism (288) providing a force against the bellow base (73) and the return spring (72) in a condensed form.

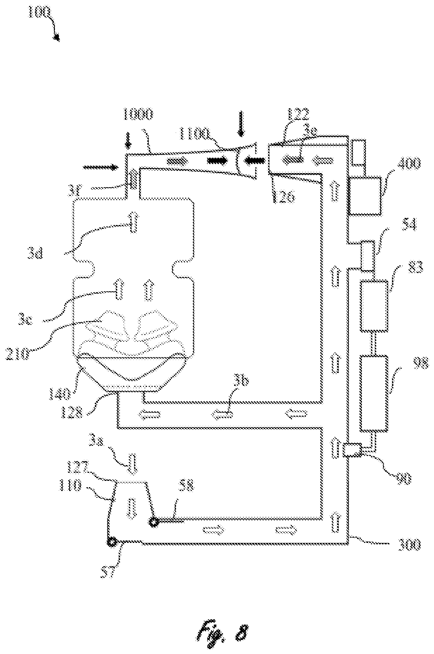

**Fig. 8** is a schematic illustration, in an out of scale manner of an example of the ear therapeutic device (100) comprising a conduit having three apertures (127, 126, 128), useful for providing pressure to the Eustachian tube (1000) from the ear connecting side and from the throat connecting side via the nasal cavity. The ear pressure equalizing means comprising at least one conduit (300) having at least one first end attachable to the ear aperture (126), at least one second end (127) connected to the mouth aperture and at least one third end (128) connected to the nose aperture. At least one first conduit end comprises an ear piece (122), and at least one second ear piece is connected to a mouth piece (110). The conduit (300) is configured to permit the passage of air from the mouth piece (110) to the ear piece (122), and to a nose piece (140) connected to the third aperture (128). The ear piece is configured to at least partially seal the external ear canal (1200), while allowing pneumatic pressure to pass through enabling application of positive or negative pressure on the ear drum (1100). The nose piece (140) comprises at least one element at least partially insertable to at least one nostril (210). The air pressure delivered through the conduit from the mouth piece along the arrows 3a arrives to the ear aperture along the arrow 3e and to the nasal cavity along the arrow 3b. In the nasal cavity the air pressure travels via (arrows 3c, 3d,) the throat to (arrow 3f) the inner opening of the Eustachian tube (1000). It is in the scope of the invention to provide pneumatic pressure to the patient nasal cavity thereby assisting in closure of the patient soft palate. The conduit (300) is further connected to a controller (98) designed to control the pneumatic pressure applied by the device (100). The controller is further connected to a release valve motor (83) and at least one release valve (54) configured to allow pressure release and pressure buildup when opened or closed accordingly. The pressured is also monitored by at least one pressure sensor (90), relaying the sensed information to such as the controller, at least one indicator, or a CRM. The device further comprises at least one vibrator (400), configured to provide oscillating vibrations to the ear by the ear piece. The vibrations can improve draining of fluids in the ear and the Eustachian tube (1000), promoting healing and /or pressure equalizing.

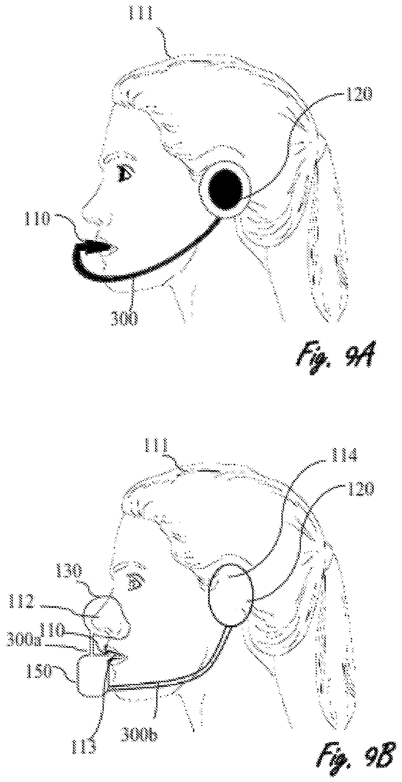

**Fig. 9A** is a schematic illustration, in an out of scale manner of an example of the present invention worn by a patient (111). A conduit (300) configured to provide pressured fluid communication, comprising at least one first aperture connected to a mouth piece (110), and at least one second aperture connected to an ear piece (120). The patient (111) blows air into the mouth piece, the air is delivered through the conduit through the ear cover into the ear, thereby applying pressure to the ear drum. The pressure applied can be positive, negative, or both.

**Fig. 9B** is a schematic illustration, in an out of scale manner of an example of the present invention worn by a patient, and connected to the patient's mouth (113), nose (112) and ear (114). A conduit (300a, 300b) configured to provide pressured fluid communication, comprising at least one first aperture connected to a mouth piece (110), and at least one second aperture connected to an ear piece (120). The patient (111) blows air into the mouth piece (110), the air is delivered through the conduit (300b) through the ear cover (120) into the ear (114), thereby applying pressure to the ear drum. Simultaneously, the pressure is applied though a third aperture, stemming from a divergence of the conduit (300a) connected to a nose cover (130) to the patient's nose (112). The pressure applied can be positive, negative, or both.

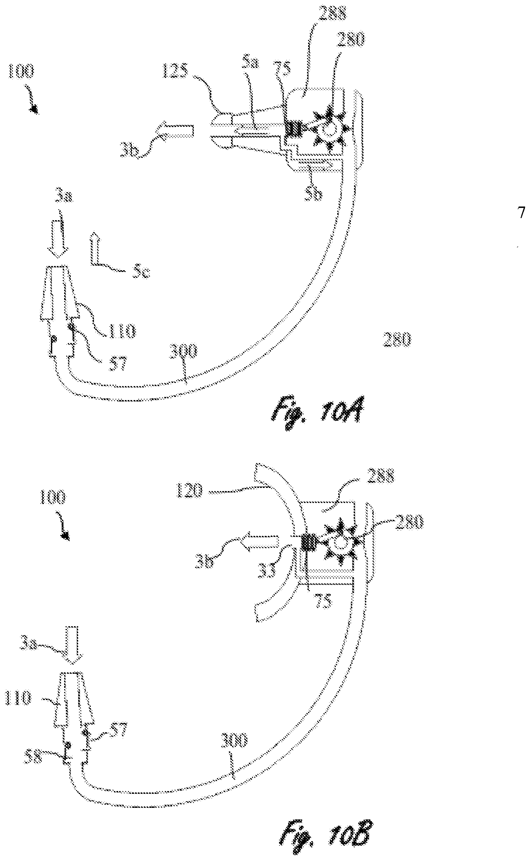

**Fig. 10A** is a schematic illustration, in an out of scale manner of an embodiment of the present invention operable by the patient's inhaling and exhaling, comprising an ear piece (120). In this embodiment, the ear piece comprises a pressure regulator, translating the fluid pressure (3a) applied by the user/ patient blowing air through the mouth piece (110), into a conduit (300). The pressure is passed through a regulating device/ gear (280) configured to translate the fluid pressure force into a rotating motion creating an oscillating translational pumping movement in the pumping sleeve (75). This pressure regulator (288) is used to enhance, reduce, or both the fluid pressure arriving (3b, 3c) at the exit port aperture (33) at the ear piece (120), at least partially insertable protruding portion (125). The returning fluid can bypass the pressure regulator as indicated by arrow 5b. The device (100) comprises at least one inhaling valve (57), and at least one exhaling valve (58), configured to allow the entry of external air when inhaling and diverting the air when exhaling into the conduit. Additionally or alternatively, the device (100) comprises at least one pressure valve further regulating the fluid pressure reaching the ear.

**Fig. 10B** is a schematic illustration, in an out of scale manner of an embodiment of the present invention, operable by the patient's inhaling / exhaling, comprising an at least partial facial cover (122). The facial cover is configured to cover at least a portion of the face, and can include covering at least apportion of the ear, mouth, nose, cheeks, jaw, forehead, head and any other feature of the head or face. In this embodiment, connected to the facial cover is a pressure regulator, translating the fluid pressure (3a) applied by the user/ patient blowing air through the mouth piece (110), into a conduit (300). The pressure is passed through a regulating device/ gear (280) configured to translate the fluid pressure force into a rotating motion creating an oscillating translational pumping movement in the pumping sleeve (75). This pressure regulator (288) is used to enhance, reduce, or both the fluid pressure arriving (3b) at the exit port aperture (33) at the facial cover (122). The device (100) comprises at least one inhaling valve (57), and at least one exhaling valve (58), configured to allow the entry of external air when inhaling and diverting the air when exhaling into the conduit.

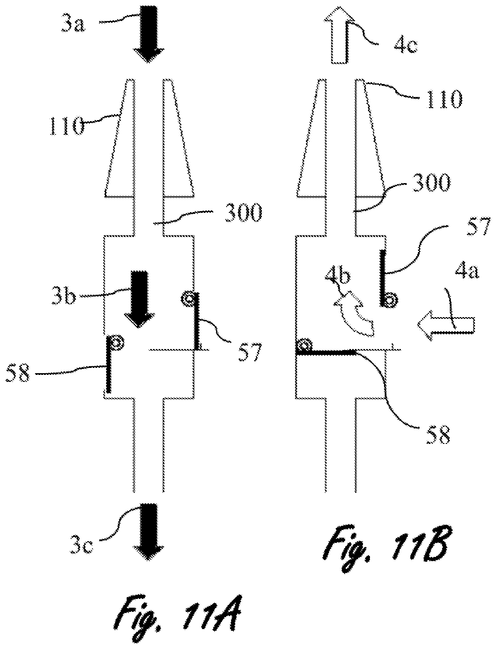

**Fig. 11A-B** is a schematic illustration, in an out of scale manner of an embodiment of a mouth piece (110) comprising inhalation and exhalation valves (57, 58). Fig. 9A illustrates that when exhaling, through the mouth piece (110) the fluid pressure is passed in the direction of the arrow 3a, and through the conduit (300). The conduit comprises a set of valves configured to divert and direct the passage of fluid as indicated by arrows 3b and 3c within the conduit. When exhaling, the exhaling valve (58) seals the exit of air from the conduit outward while permitting the fluid pressure to advance in the direction or the arrow. The inhalation valve (57) remains closed, sealing the exit of air from the conduit. When inhaling, as illustrated in Fig 9B, the inhalation valve (57) swings open to allow the entry of external air through the conduit (300) to the mouthpiece (110) in the direction of the arrows 4a, 4b, 4c. The exhalation valve (58) blocks the passage of air in the conduit, thereby blocking the formation of negative pressure in the ear/ nose.

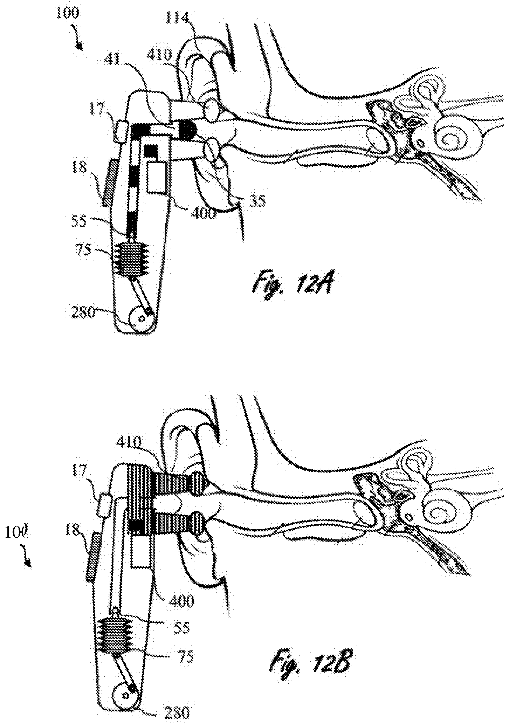

**Fig. 12A-B** are schematic illustrations, in an out of scale manner of different examples of the invention comprising an ear vibrator. The ear therapeutic device (100), comprising at least one fluid pressure pump (75) regulated by a pressure regulator (280) gear connected to a motor, configured to administer a plurality of protocols and sub protocols of pressure (continuous, oscillating, intermittent or any combination thereof). The fluid pressure passes through at least one conduit (41) and operative to inflate an inflatable balloon or flexible membrane (35) at the interface of the ear therapeutic device (100) with the ear external canal. The device further comprises at least one vibrator (400) and at least one vibrating element (410), interfacing with the ear (114). The device (100) is configured to administrate protocols and/ or sub protocols of oscillating vibrations to the ear (114) thereby assisting fluid draining from the ear portions such as the Eustachian tube and the middle ear. Additionally or alternatively, the vibrations are administered alone or simultaneously with fluid pressure protocols or sub protocols. The device further comprises at least one pressure valve configured to regulate the pressure applied (55). Additionally or alternatively, the device comprises a user interface including a pressure pulses regulating turnable button (18), and at least one on / off button (17).

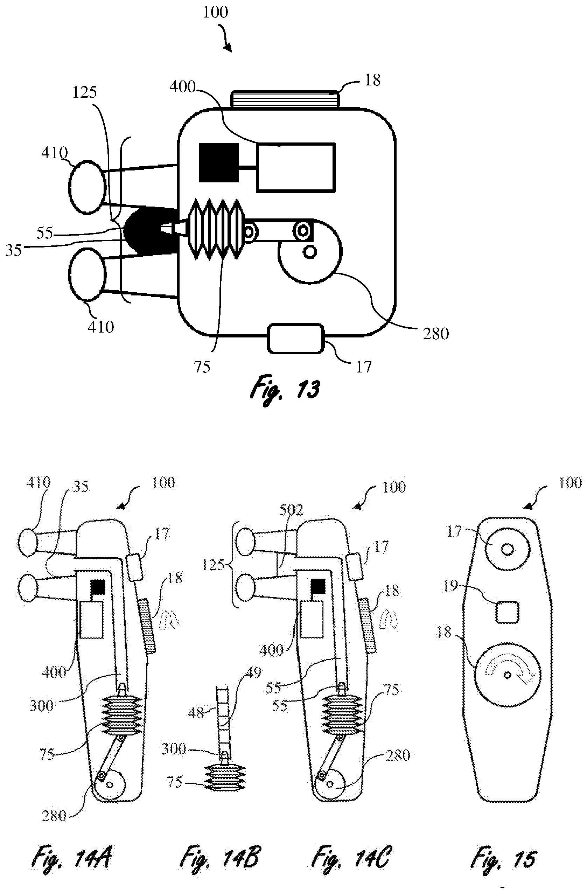

**Fig. 13** is a schematic illustration, in an out of scale manner of an example of the top view of the invention (100), comprising pneumatic pressure means (500) and vibration means (400) as part of the ear piece (125). The pressure means comprise at least one pressure pump (75) providing pneumatic pressure configured to inflate/ deflate at least one inflatable balloon (35). The pressure can be adjusted by at least one pressure valve (55) attached at the interface of the pneumatic pump (35) and the inflatable balloon (35). The vibration means comprise at least one vibrator (400) and at least one vibrating element (410). The device (100) further comprises at least one pressure pulse regulator (18) adjustable by the user, and at least one on/off button (17). The Pump is connected by a gear (280) to a motor. Additionally or alternatively, the pump can be operated manually, automatically or both.

**Fig. 14 A-C** are schematic illustrations, in an out of scale manner of different examples of the ear therapeutic device comprising an inflatable membrane/ balloon (35) configured to administer pressure to at least a portion of the patients' ear, and oscillating vibration providing means. The ear therapeutic device (100), comprising at least one fluid pressure pump (75) regulated by a gear (280) configured to translate the turning movement of a motor to a pulsating motions, configured to administer a plurality of protocols and sub protocols of pressure (continuous, oscillating, intermittent, pulsating or any combination thereof). The fluid pressure passes through at least one conduit (300) and operative to inflate an inflatable balloon or flexible membrane (35) at the interface of the ear therapeutic device (100) with the ear external canal. The ear piece (125) comprises both a pressure administrating inflatable membrane (35) and at least one vibrating element (410). The device further comprises at least one vibrator (400) and at least one vibrating element (410), interfacing with the ear (114). The device (100) is configured to administrate protocols and/ or sub protocols of oscillating vibrations to the ear (114) thereby assisting fluid draining from the ear portions such as the Eustachian tube and the middle ear. Additionally or alternatively, the vibrations are administered alone or simultaneously with fluid pressure protocols or sub protocols. The device further comprises at least one pressure valve configured to regulate the pressure applied (55). Additionally or alternatively, the device comprises a user interface including a pressure pulses regulating button (18), and at least one on / off button (17). **Fig 12B** illustrates the conduit (300) connected to a pump (75) via at least one valve, providing pulsating pneumatic pressure. As a non-limiting example, the pressure is of a specific value at area 49, and at a lower value at area 48.

**Fig. 15** is a schematic illustration, in an out of scale manner of an example of the invention (100) in a side view, showing an example of a user interface. In this example the user interface comprises a pressure regulating button (18), an on/ off switch (17) and a program selector (19) for selecting a treatment protocol.

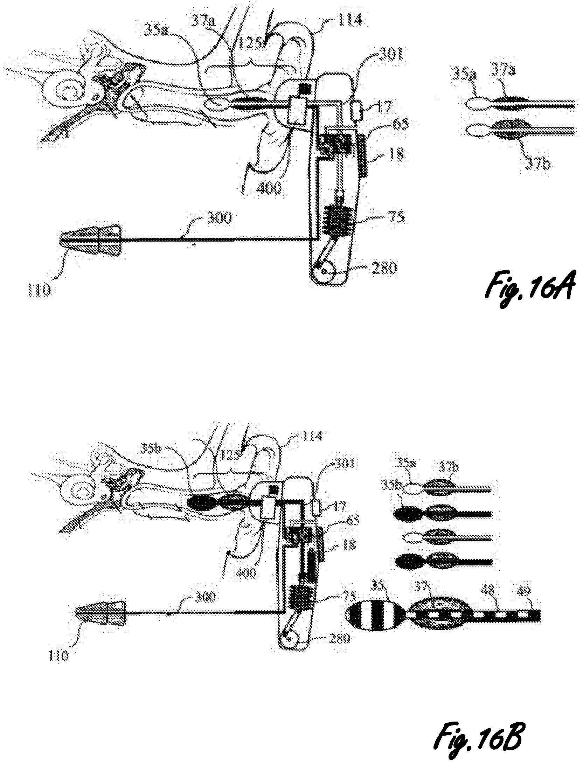

**Fig. 16 A-B** is a schematic illustration, in an out of scale manner of an embodiment of the ear therapeutic device (100) in a side view. In this embodiment, the ear device (100) comprises two conduits (300, 301) configured to control and regulate pressure administered to the ear. One first conduit (300) comprising at least two end apertures. One first end connected to a mouth piece (110) configured to allow fluid connection applied by a user's mouth via the mouth piece (110), through the conduit (300) to an inflatable membrane (37a, b) connected to at least one second conduit aperture. The inflatable membrane (37a, b) is configured to at least partially seal the external ear auditory canal. The user can regulate the closure of the external auditory canal by such as inhaling, exhaling, forcibly blowing air, through the mouth piece (100), thereby inflating and/ or deflating the membrane (37a, b). A second conduit (301) comprising at least ends, each having an aperture, configured to allow fluid connection therebetween. At least one first end connected to a pressure means such as a pump, bellow, piston, operational manually, automatically or both. In this embodiment a motor is connected to a gear (280) translating the rotational force to a pulsating force activating a pump (75) configure to inflate/ deflate an inflatable balloon or flexible membrane (35a, b), thereby administrating pressure to the ear. The ear piece (125) comprises at least two inflatable membranes (37a, b, 35a, b) at least partly inserted into the external auditory canal. The first membrane (35) inserted at least partly within the ear and is configured to be in at least an inflated configuration (35b) and deflated configuration (35a) and any partly inflated configuration. The user can regulate the pressure administered to the ear by controlling the motor velocity by at least one interface button (18), an on/ off button (17). Importantly, the pressure applied by the first membrane (35) is further regulated by at least partly sealing the ear external canal by the second membrane (37), inflated / deflate by the user. When the second membrane is deflated (37a) the first membrane is inflated (35b) with less resistance since the surrounding air can be pushed out of the ear to the external environment. When the second membrane is inflated (37b), thereby sealing the ear canal, the pressure administered to the ear by inflating the first membrane (35b) is greater than when the ear canal is open. The air pressure administered by the mouthpiece (110) can also be regulated by a pressure control device (65) connected to at least one conduit (300, 301) providing both on/off switch and pressure level control, responsive to the user's inhaling, exhaling, forcefully breathing or blowing air through the mouthpiece (110). The ear device (100) further comprises at least one vibration providing means such as a vibrator (400), configured to provide oscillating vibrations to the ear through at least a portion of the ear piece (125). **Fig. 16A** illustrates a configuration where one membrane (35a) is at least partly inserted into the external auditory canal, and presented in a deflated configuration. A second membrane (37a) is at least partly inserted into the external ear canal entrance, in a deflated configuration (37a), external to the first membrane (35a). **Fig 16B** illustrates a configuration where the external membrane is inflated (37b) thereby at least partly sealing the ear external auditory canal. This configuration enables administrating pneumatic pressure by inflating/ deflating a second membrane (35a, b) located at least partly within the external ear canal between the ear drum and the first membrane (37).

Additionally or alternatively, when the ear canal is sealed, by an inflated sealing membrane (37b) a plurality of pressure and or vibration protocols and sub protocols be administered to the patient's ear, including alternating pressure waves (48,49) provided by inflating and/or deflating the inner membrane (35).

Additionally or alternatively, the inner membrane (35) and or the sealing membrane (37) is connectable to at least one pressure means such as a pump, piston, bellow, compressor, vent, a user's respiratory system, a flexible bag and etc.

Additionally or alternatively, the ear device (100), at least one pressure means, at least one conduit, at least one inflatable/ deflatable membrane, at least one pressure control device, and any combination thereof, comprises at least one valve configured to regulate the pressure administered by the ear device.

Additionally or alternatively, the ear device (100) comprises at least one pressure sensor configured to sense the pressure administered by the pressure means. Additionally or alternatively, the sensor relays information to at least one indicator, selected from a group consisting of visual, auditable, sensible and any combination thereof. Additionally or alternatively, the sensor relays sensed information to a selected from a group consisting of: a CRM, a remote computer, a remote control, an emergency shut off system, a pressure valve, a pressure control device, at least one indicator, or any combination thereof.

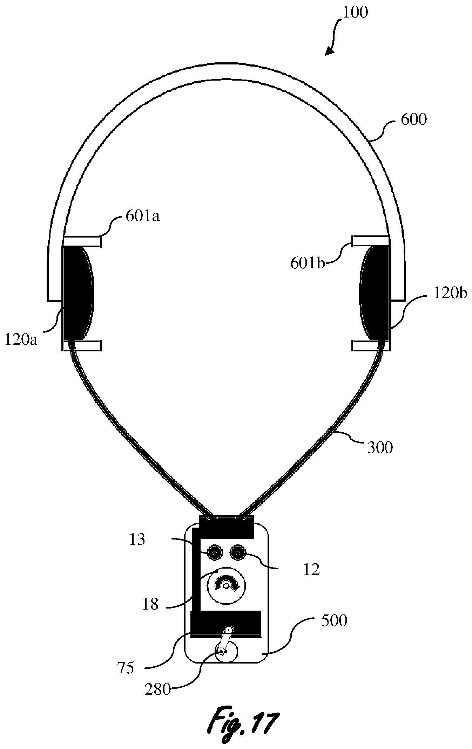

**Fig. 17** is a schematic illustration, in an out of scale manner of an example of the invention comprising a remote control system. In this example a headset (600) fitted to reside on a patient head, is provided, comprising at least one placement (601a, 601b) configured to house at least one ear piece (120a, 120b). The ear piece is in fluid communication with at least one pressure equalizing means (500) such as a pneumatic pump (75) by a conduit (300), having at least one aperture for the entry of fluid and at least one aperture connected to at least one ear piece (120). The pneumatic pump (75) is connected to a motor providing torque translated to a pulsating movement by a gear (280) element. The pump, motor and operational buttons (13, 12) are located remote from the ear, in a remote control station/ hand held device.

## Claims

1. An ear therapeutic device ETD (100) comprising:
a. middle ear ambient pressure equalizing means (500) comprising at least one conduit (300) comprising at least one first end (127) configured for receiving an airflow blown thereinto and at least one second end (126) provided with an ear piece (120) configured for external attachment to the ear aperture being in a fluid communication therebetween;
b. a vibrator (400) inducing vibrations propagating into said patient Eustachian tube (1000);
c. at least one inhaling valve (57) and at least one exhaling valve (58) configured for to allow the entry of external air when inhaling and diverting the air when exhaling into the conduit;
d. a mouth piece (110) connected to said first end and conducting said airflow blown by a patient into said conduit (300); and
wherein said ear piece (120) comprises
a pressure regulator (65; 288) of said airflow at said ear piece (120) configured for regulating airflow pressure at an exit port aperture (33) of said ear piece (120), and comprising a regulating gear (280) configured to translate a fluid pressure force into rotating motion creating an oscillating translational pumping movement in a pumping sleeve (75):
and
a partially insertable protruding portion (125).

2. The ETD according to claim 1, wherein at least a portion of said conduit is made of a material selected from the group consisting of: a flexible material, at least partially transparent material, a sterilizable material, a disposable material, and any combination thereof.

3. The ETD according to claim 1, wherein said ETD further comprises at least one valve configured to regulate a pressure administered by said pressure equalizing means.

4. The ETD according to claim 1, wherein said conduit comprises at least one pressure regulating valve (57) operative by said patient inhaling, exhaling, or both.

5. The ETD according to claim 1, wherein said device further comprises at least one operating system configured to control means selected from the group consisting of: one or more said vibration means, one or more said pressure equalizing means, one or more said valves, and any combination thereof.

6. The ETD according to claim 1, wherein said ETD comprises a pressure sensor (90).

## Patentansprüche

1. Ein Ohrtherapiegerät ETD (100), umfassend:
a. ein Mittelohr-Umgebungsdruck-Ausgleichsmittel (500), das mindestens eine Leitung (300) umfasst, die mindestens ein erstes Ende (127), das zum Aufnehmen eines darin eingeblasenen Luftstroms konfiguriert ist, und mindestens ein zweites Ende (126) umfasst, das mit einem Ohrstück (120) versehen ist, das konfiguriert für eine externe Befestigung an der Ohröffnung, die in einer Fluidverbindung dazwischensteht;
b. einen Vibrator (400), der Schwingungen induziert, die sich in die Eustachische Röhre (1000) des Patienten ausbreiten;
c. mindestens ein Einatmungsventil (57) und mindestens ein Ausatmungsventil (58), die konfiguriert sind, um den Eintritt von Außenluft beim Einatmen zuzulassen und die Luft beim Ausatmen in die Leitung umzuleiten;
d. ein Mundstück (110), das mit dem ersten Ende verbunden ist und den von einem Patienten geblasenen Luftstrom in die Leitung (300) leitet; und
wobei das Ohrstück (120) umfasst
einen Druckregler (65; 288) des Luftstroms an dem Ohrstück (120), der zum Regulieren des Luftstromdrucks an einer Austrittsöffnung (33) des Ohrstücks (120) konfiguriert ist und ein Regulierzahnrad (280) umfasst, das zum Verschieben konfiguriert ist eine Fluiddruckkraft in eine Drehbewegung, die eine oszillierende Pumpbewegung in einer Pumphülse (75) erzeugt; und
einen teilweise einführbaren vorstehenden Abschnitt.

2. Das ETD nach Anspruch 1, wobei mindestens ein Teil der Leitung aus einem Material hergestellt ist, das aus der Gruppe ausgewählt ist, die besteht aus: einem flexiblen Material, einem mindestens teilweise transparenten Material, einem sterilisierbaren Material, einem Einwegmaterial und einer beliebigen Kombination davon.

3. Das ETD nach Anspruch 1, wobei die ETD ferner mindestens ein Ventil umfasst, das konfiguriert ist, um einen Druck zu regulieren, der durch die Druckausgleichseinrichtung ausgeübt wird.

4. Das ETD nach Anspruch 1, wobei die Leitung mindestens ein Druckregelventil (57) umfasst, das durch Einatmen, Ausatmen oder beides des Patienten betätigt wird.

5. Das ETD nach Anspruch 1, wobei die Vorrichtung ferner mindestens ein Betriebssystem umfasst, das zum Steuern von Mitteln konfiguriert ist, die ausgewählt sind aus der Gruppe bestehend aus: einem oder mehreren der Vibrationsmittel, einem oder mehreren der Druckausgleichsmittel, einem oder mehreren der Ventile, und jede Kombination davon.

6. Das ETD nach Anspruch 1, wobei das ETD einen Drucksensor (90) umfasst.

## Revendications

1. Dispositif thérapeutique auriculaire ETD (100) comprenant :
a. un moyen d'égalisation de la pression ambiante de l'oreille moyenne (500) comprenant au moins un conduit (300) comprenant au moins une première extrémité (127) configurée pour recevoir un flux d'air soufflé dans celui-ci et au moins une seconde extrémité (126) munie d'un embout (120) configuré pour une fixation externe à l'ouverture de l'oreille étant en communication fluidique entre elles ;
b. un vibrateur (400) induisant des vibrations se propageant dans ladite trompe d'Eustache (1000) du patient ;
c. au moins une soupape d'inspiration (57) et au moins une soupape d'expiration (58) configurées pour permettre l'entrée d'air extérieur lors de l'inspiration et détourner l'air lors de l'expiration dans le conduit ;
d. un embout buccal (110) relié à ladite première extrémité et conduisant ledit flux d'air soufflé par un patient dans ledit conduit (300) ; et
dans lequel ledit embout (120) comprend
un régulateur de pression (65 ; 288) dudit écoulement d'air au niveau dudit embout (120) configuré pour réguler la pression d'écoulement d'air au niveau d'une ouverture d'orifice de sortie (33) dudit embout (120), et comprenant un engrenage de régulation (280) configuré pour translater une force de pression de fluide en mouvement de rotation créant un mouvement de pompage oscillant dans un manchon de pompage (75) ; et
une partie saillante partiellement insérable.

2. Le ETD selon la revendication 1, dans lequel au moins une partie dudit conduit est constituée d'un matériau choisi dans le groupe constitué par : un matériau flexible, un matériau au moins partiellement transparent, un matériau stérilisable, un matériau jetable et toute combinaison de celui-ci.

3. Le ETD selon la revendication 1, dans lequel ledit ETD comprend en outre au moins une soupape configurée pour réguler une pression administrée par lesdits moyens d'égalisation de pression.

4. Le ETD selon la revendication 1, dans lequel ledit conduit comprend au moins une soupape de régulation de pression (57) actionnée par ledit patient inhalant, expirant, ou les deux.

5. Le ETD selon la revendication 1, dans lequel ledit dispositif comprend en outre au moins un système d'exploitation configuré pour contrôler des moyens choisis dans le groupe constitué de : un ou plusieurs lesdits moyens de vibration, un ou plusieurs lesdits moyens d'égalisation de pression, une ou plusieurs desdites soupapes, et toute combinaison de ceux-ci.

6. Le ETD selon la revendication 1, dans lequel ledit ETD comprend un capteur de pression (90).
